(19) Europäisches Patentamt
European Patent Office
Office européen des brevets

(11) Veröffentlichungsnummer: **0 318 891 B1**

(12) **EUROPÄISCHE PATENTSCHRIFT**

(45) Veröffentlichungstag der Patentschrift: **02.01.92**

(51) Int. Cl.[5]: **A61K 31/385**, A61K 9/08, A61K 47/00

(21) Anmeldenummer: **88119790.9**

(22) Anmeldetag: **28.11.88**

Die Akte enthält technische Angaben, die nach dem Eingang der Anmeldung eingereicht wurden und die nicht in dieser Patentschrift enthalten sind.

(54) **Injizierbare Lösung des Thioctsäuresalzes mit Trometamol und/oder basischen Aminosäuren.**

(30) Priorität: **04.12.87 DE 3741152**

(43) Veröffentlichungstag der Anmeldung:
**07.06.89 Patentblatt 89/23**

(45) Bekanntmachung des Hinweises auf die Patenterteilung:
**02.01.92 Patentblatt 92/01**

(84) Benannte Vertragsstaaten:
**AT BE CH DE ES FR GB GR IT LI LU NL SE**

(56) Entgegenhaltungen:

| | |
|---|---|
| **EP-A- 0 093 999** | **DE-A- 1 695 358** |
| **DE-A- 2 629 845** | **DE-B- 1 047 991** |
| **DE-B- 1 056 784** | **DE-B- 1 095 991** |
| **DE-B- 1 617 740** | **FR-A- 2 110 465** |
| **FR-U- 4 630** | **GB-A- 1 504 767** |

(73) Patentinhaber: **ASTA Pharma AG**
**Weismüllerstrasse 45**
**W-6000 Frankfurt am Main 1(DE)**

(72) Erfinder: **Hettche, Helmut, Dr.**
**Martinstrasse 23**
**W-6057 Dietzenbach(DE)**
Erfinder: **Muckenschnabel, Reinhard, Dr.**
**Odenwaldring 5**
**W-6050 Offenbach(DE)**

**EP 0 318 891 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine injizierbare Lösung des Thioctsäuresalzes mit Trometamol und/oder mit basischen Aminosäuren. Das salzbildende Säuren/Basen-Paar liegt hierbei im molaren Verhältnis Thioctsäure: Trometamol = 1 : 1,1 bis 3.2 und/oder Thioctsäure: basischer Aminosäure = 1 1,1 bis 2 vor.

Thioctsäure (α-Liponsäure) ist chemisch eine 1,2-Dithiacyclopentan-3-valeriansäure:

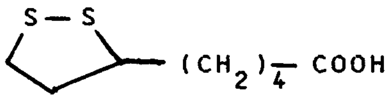

Thioctsäure ist Bestandteil des Zellstoffwechsels und wird daher in vielen Pflanzen und tierischen Organismen gefunden. Sie wirkt als eines der Coenzyme bei der oxydativen Decarboxylierung von Pyruvat und anderen α-Ketosäuren.

Thioctsäure wird seit längerer Zeit bei verschiedenen Erkrankungen eingesetzt, so u.a. bei Lebererkrankungen, bei Leberschädigung durch Pilzvergiftung sowie bei diabetischer und alkoholischer Polyneuropathie, einer mit Stoffwechselerkrankungen einhergehenden Veränderung peripherer Nerven.

Bei der Therapie mit Thioctsäure wird, vornehmlich im Anfangsstadium der Behandlung, die Injektionslösung eingesetzt.

Thioctsäure hat indessen den Nachteil, daß sie in Wasser und in den üblichen, zur Herstellung von Injektionsprodukten verwendeten Lösungsmitteln nur wenig löslich ist und außerdem chemisch nicht ausreichend stabil ist, da die S-S-Bindung verhältnismäßig leicht gespalten wird, wobei freie Ionen entstehen, die im Laufe der Zeit zu innermolekularen Polymerisationen führen. Man verwendet gewöhnlich die Salze dieser Säuren, da deren Löslichkeit besser ist.

Verschiedene Salzbildner der Thioctsäure führen bei der Injektion der Lösung zu Unverträglichkeiten, wie z.B. Kopfdruck, Flush, Atembeklemmung und brennenden Schmerzen an der Injektionsstelle.

Als weiteren Nachteil zeigen Injektionslösungen mit verschiedenen Salzbildnern nur eine geringe Stabilität, da sie zur Bildung von Trübungen und Fällungen neigen.

Nach einer allgemeinen medizinisch-pharmazeutischen Regel müssen Lösungen, die im Laufe der Lagerung trübe werden, als zersetzt gelten, und diese dürfen nicht mehr angewendet werden.

Es war daher Aufgabe der Erfindung, gut verträgliche injizierbare Lösungen von Thioctsäuresalzen bereitzustellen, wobei gleichzeitig die Lagerstabilität der Lösungen erhalten bleibt.

Es wurde gefunden, daß injizierbare Lösungen mit den gewünschten verbesserten Eigenschaften durch Einsatz des Thioctsäuresalzes mit Trometamol und/oder mit basischen Aminosäuren hergestellt werden können, wobei das salzbildende Säure-Base-Paar im molaren Verhältnis Thioctsäure: Trometamol = 1 : 1,1 bis 3,2 und/oder Thioctsäure: basischer Aminosäure = 1 : 1,1 bis 2 vorliegt. Das Salz der Thioctsäure mit Trometamol (2-Amino-2-hydroxymethylpropan-1,3-diol) im molaren Verhältnis 1 : 1 und dessen Herstellung ist in der DE-OS 1695 358 beschrieben. Es ist ein gelbes kristallines Pulver mit einem Fp von 120 °C, das in Wasser und heißem Alkohol stark löslich ist. Eine 0,1 M-wässrige Lösung hat einen nahezu neutralen pH-Wert von 6,5. Trometamol ist ein bekanntes pharmazeutisches Mittel, das sowohl als Plasmapuffer als auch als intrazellulärer Puffer wirkt.

Salze der Thioctsäure mit Aminosäuren sind aus dem französischen Patent No. 4.630 M sowie aus den spanischen Patenten 313 056 und 365 246 bekannt. Die Salze werden durch äquimolare Reaktionen von Thioctsäure und Aminosäuren hergestellt, so daß deren 1-%ige wässrige Lösungen einen neutralen pH-Wert zeigen.

Nach dem Stand der Technik (DE-OS 1056 784, DE-OS 1047 991) ist es bekannt, lagerstabile injizierbare Lösungen von Salzen der Thioctsäure bereitzustellen, die einen physiologischen oder schwach alkalischen pH-Wert aufweisen. Im Gegensatz zu den bekannten injizierbaren Lösungen mit den nachteiligen Eigenschaften zeigen die erfindungsgemäß hergestellten gut verträglichen Lösungen einen basischen pH-Wert von 7,6 bis ca. 8,8.

Gegebenenfalls kann auch noch die Zugabe einer Base wie zum Beispiel NaOH erfolgen, um den basischen pH-Wert zu erreichen. Die gute Verträglichkeit ist überraschend, da bei dem bekannten Salz der Thioctsäure mit Ethylendiamin eine Erhöhung des Aminoanteils demgegenüber in der injizierbaren Verabreichung zu einer schlechten Verträglichkeit führt.

Eine bevorzugte Ausführungsform ist die Bereitstellung einer injizierbaren Lösung bestehend aus dem Thioctsäuresalz mit Trometamol und/oder mit basischen Aminosäuren, wobei das Säure-Basen-Paar im molaren Verhältnis Thioctsäure : Trometamol = 1 : 1,4 bis 2,8 und/oder Thioctsäure : basischer Aminosäure = 1 : 1,2 bis 1,4 vorliegt. Eine besonders bevorzugte Ausführungsform ist eine injizierbare Lösung, bei der das molare Verhältnis von Thioctsäure: Trometamol = 1 : 2,4 beträgt. Bei dem Salz der Thioctsäure mit einer

basischen Aminosäure ist eine bevorzugte Ausführungsform das Salz mit der basischen Aminosäure L-Lysin oder L-Arginin. Weiterhin kann in gleicher Weise das entsprechende Mischsalz bestehend aus Thioctsäure : Trometamol : basischer Aminosäure eingesetzt werden. Die Thioctsäure kann entweder in der racemischen D,L-Form oder als Enantiomeres vorliegen.

Zur Herstellung der erfindungsgemäßen injizierbaren Lösungen können gegebenenfalls weitere übliche pharmazeutische Hilfsstoffe wie zum Beispiel Benzylalkohol, Propylenglykol, Ethanol, Glycerol, Sorbitol, Mannitol, flüssige Polyethylenglycole (Typen 200-600) Tetraglykol (Glycofurol$^R$) oder Butylenglycol (1,3-Butandiol) verwendet werden.

Die Lösungen werden hergestellt, indem Thioctsäure und die basische Aminosäure und/oder Trometamol in Wasser bis zur Lösung gerührt werden. Die Lösung wird anschließend mit Wasser auf das erforderliche Volumen aufgefüllt, filtiert, in Ampullen gefüllt und mit gespanntem Wasserdampf bei 121 °C sterilisiert. Als weiteren Vorteil zeigt die Erfindung, daß die Lösung nach Abfüllung in Ampullen auf diese Weise sterilisiert werden kann, was die Einhaltung der umständlichen und kostenintensiven aseptischen Abfüllung entbehrlich und das Herstellverfahren damit sicherer macht.

Die folgenden Beispiele erläutern die Erfindung:

250 g Thioctsäure werden zusammen mit 352,3 g Trometamol in 9 Liter Wasser für Injektionszwecke unter Rühren gelöst. Die Lösung wird mit Wasser für Injektionszwecke auf 10 Liter aufgefüllt und anschließend durch ein Membranfilter der Porenweite 0,2 µm mit Glasfaservorfilter filtriert. Das Filtrat wird zu 2 ml in 2 ml-Ampullen abgefüllt.

Die Ampullen werden 15 Minuten lang im gespannten Wasserdampf bei 121 °C sterilisiert.

Eine Ampulle enthält in 2 ml Injektionslösung 50 mg Thioctsäure als Trometamolsalz.

250 g Thioctsäure werden zusammen mit 278,6 g L-Lysin-Monohydrat in 9 Liter Wasser für Injektionszwecke unter Rühren gelöst. Die Lösung wird mit Wasser für Injektionszwecke auf 10 Liter aufgefüllt und anschließend durch ein Membranfilter der Porenweite 0,2 µm mit Glasfaservorfilter filtriert. Das Filtrat wird zu 2 ml in 2 ml-Ampullen abgefüllt. Die Ampullen werden 15 Minuten lang im gespannten Wasserdampf bei 121 °C sterilisiert.

Eine Ampulle enthält in 2 ml Injektionslösung 50 mg Thioctsäure als L-Lysinsalz.

## Patentansprüche

### Patentansprüche für folgende Vertragsstaaten : AT, BE, CH, DE, FR, GB, IT, LI, LU, NL, SE

**1.** Injizierbare Lösung des Thioctsäuresalzes mit Trometamol und/oder basichen Aminosäuren, dadurch gekennzeichnet, daß das salzbildende Säure/Basen-Paar im molaren Verhältnis Thioctsäure : Trometamol = 1 : 1,1 bis 3,2 und/oder Thioctsäure : basischer Aminosäure = 1 : 1,1 bis 2 vorliegt sowie mit gegebenenfalls anderen üblichen pharmazeutischen Hilfsstoffen.

**2.** Injizierbare Lösung nach Anspruch 1, dadurch gekennzeichnet, daß das salzbildende Säuren/Basen-Paar im molaren Verhältnis Thioctsäure : Trometamol = 1 : 1,4 bis 2,8 vorliegt.

**3.** Injizierbare Lösung nach Anspruch 1, dadurch gekennzeichnet, daß das salzbildende Säuren/Basen-Paar im molaren Verhältnis Thioctsäure : basischer Aminosäure = 1 : 1,2 bis 1,4 vorliegt.

**4.** Injizierbare Lösung nach den Ansprüchen 1 und 3, dadurch gekennzeichnet, daß die basiche Aminosäure L-Lysin oder L-Arginin ist.

**5.** Injizierbare Lösung nach den Ansprüchen 1 bis 4, dadurch gekennzeichnet, daß das salzbildende Säure/Basen-Paar aus Thioctsäure/Trometamol und basischer Aminosäure hergestellt wird.

**6.** Verfahren zur Herstellung einer injizierbaren Lösung nach den Ansprüchen 1 bis 5, dadurch gekennzeichnet, daß Thioctsäure zusammen mit Trometamol und/oder einer basischen Aminosäure in Wasser für Injektionszwecke unter Rühren gelöst, durch ein Membranfilter der Porenweite 0,2 µm filtriert und in Ampullen abgefüllt und abschließend 15 Minuten im gespannten Wasserdampf bei 121 °C sterilisiert wird.

### Patentanspruch für folgende Vertragsstaaten : ES, GR

**1.** Verfahren zur Herstellung einer injizierbaren Lösung des Thioctsäuresalzes mit Trometamol und/oder basischen Aminosäuren, dadurch gekennzeichnet, daß das salzbildende Säure/Basen-Paar im molaren Verhältnis Thioctsäure : Trometamol = 1 : 1,1 bis 3,2 und/oder Thioctsäure : basische Aminosäure = 1 : 1,1 bis 2 in Wasser für Injektionszwecke unter Rühren gelöst, durch ein Membranfilter der Porenweite 0,2 µm filtriert, in Ampullen abgefüllt und anschließend 15 Minuten im gespannten Wasserdampf bei 121 °C sterilisiert wird.

**Claims**

**Claims for the following Contracting States : AT, BE, CH, DE, FR, GB, IT, LI, LU, NL, SE**

1. Injectable solution of the thioctic acid salt of trometamol and/or basic amino acids, characterised in that the salt forming acid/base pair is present in a molar ratio of thioctic acid : trometamol = 1 : 1.1 to 3.2 and/or thioctic acid : basic amino acid = 1 : 1.1 to 2, and optionally together with other conventional pharmaceutical auxiliary substances.

2. Injectable solution according to Claim 1, characterised in that the salt forming acid/base pair is present in a molar ratio of thioctic acid : trometamol = 1 : 1.4 to 2.8.

3. Injectable solution according to Claim 1, characterised in that the salt-forming acid/base pair is present in a molar ratio of thioctic acid : basic amino acid = 1 : 1.2 to 1.4.

4. Injectable solution according to Claims 1 and 3, characterised in that the basic amino acid is L-lysine or L-arginine.

5. Injectable solution according to Claims 1 to 4, characterised in that the salt-forming acid/base pair is prepared from thioctic acid/trometamol and basic amino acid.

6. A process for the preparation of an injectable solution according to Claims 1 to 5, characterised in that thioctic acid together with trometamol and/or a basic amino acid is dissolved in water with stirring for purposes of injection, filtered through a membrane filter of pore size 0.2 µm and filled into ampoules and finally sterilized in superheated steam at 121 °C for 15 minutes.

**Claim for the following Contracting States : ES, GR**

1. A process for the preparation of an injectable solution of the thioctic acid salt of trometamol and/or basic amino acids, characterised in that the salt forming acid/base pair in a molar ratio of thioctic acid : trometamol = 1 : 1.1 to 3.2 and/or thioctic acid : basic amino acid = 1 : 1.1 to 2 is dissolved in water with stirring for purposes of injection, filtered through a membrane filter of pore size 0.2 µm, filled into ampoules and finally sterilized in superheated steam at 121 °C for 15 minutes.

**Revendications**

**Revendications pour les Etats contractants suivants : AT, BE, CH, DE, FR, GB, IT, LI, LU, NL, SE**

1. Solution injectable du sel de l'acide thioctique avec le trometamole et/ou des acides aminés basiques, caractérisée en ce que la paire acide/bases salifiable y est présente dans un rapport molaire acide thioctique : trometamole = 1 : 1,1 à 3,2 et/ou acide thioctique : acide aminé basique = 1 : 1,1 à 2 et, éventuellement, avec d'autres adjuvants pharmaceutiques usuels.

2. Solution injectable selon la revendication 1, caractérisée en ce que la paire acides/bases salifiable y est présente dans un rapport molaire acide thioctique: trometamole = 1 : 1,4 à 2,8.

3. Solution injectable selon la revendication 1, caractérisée en ce que la paire acides/bases salifiable y est présente dans un rapport molaire acide thioctique: acide aminé basique = 1 : 1,2 à 1,4.

4. Solution injectable selon les revendications 1 et 3, caractérisée en ce que l'acide aminé basique est la L-lysine ou la L-arginine.

5. Solution injectable selon l'une quelconque des revendications 1 à 4, caractérisée en ce que la paire acide/bases salifiables est préparée à partir d'acide thioctique/trometamole et d'acide aminé basique.

6. Procédé de préparation d'une solution injectable selon l'une quelconque des revendications 1 à 5, caractérisé en ce qu'on dissout sous agitation, dans de l'eau pour injection, de l'acide thioctique, en même temps que du trometamole et/ou un acide aminé basique, on filtre à travers une membrane filtrante de 0,2 µm de largeur de pores et on remplit des ampoules, puis on stérilise à 121 °C pendant 15 mn dans de la vapeur d'eau sous pression.

**Revendication pour les Etats contractants suivants : ES, GR**

1. Procédé de préparation d'une solution injectable du sel d'acide thioctique avec le trometamole et/ou des acides aminés basiques, caractérisé en ce qu'on dissout sous agitation, dans de l'eau pour injection, la paire acide/bases salifiable dans un rapport molaire acide thioctique : trometamole = 1 : 1,1 à 3,2 et/ou acide thioctique : acide aminé basique = 1 : 1,1 à 2,

on filtre à travers une membrane filtrante de 0,2 µm de largeur de pores, on remplit des ampoules, puis on stérilise à 121 °C pendant 15 mn dans de la vapeur d'eau sous pression.